# EUROPEAN PATENT APPLICATION

(11) **EP 2 145 951 A1**
(43) Date of publication of application: **20.01.2010**
(21) Application number: 08752523.4
(22) Date of filing: 09.05.2008
(51) Int. Cl.: C12N 5/06, A61L 27/00

(54) **METHOD OF DEGRADING BIOLOGICAL TISSUE**

(30) Priority: 11.05.2007 JP 2007126968
(71) Applicant: Olympus Corporation, Tokyo 151-0072 (JP); Cytori Therapeutics, Inc., San Diego, California 92121 (US)
(72) Inventor: TSUCHIDA, Hiroki, Tokyo 151-0072 (JP)
(74) Representative: von Hellfeld, Axel
(86) International application number: PCT/JP2008/058640
(87) International publication number: WO 2008/140044

(57) **Abstract**

A biological tissue is decomposed in a short time, and cells are obtained at a high yield without interfering with the health of cells obtained through the decomposition. There is provided a method for decomposing biological tissue which decomposes a biological tissue under an action of a digestive enzyme, comprising: a decomposition starting step S32 of starting the decomposition of the biological tissue by immersing the biological tissue in a digestive juice having the digestive enzyme mixed in a solvent; and an enzyme concentration reduction step S31 of reducing a concentration of the digestive enzyme in the digestive juice at a predetermined timing after the start of the decomposition.

## Description

### Technical Field

The present invention relates to a method for decomposing biological tissue.

### Background Art

Conventionally, methods for decomposing biological tissue are known in which a biological tissue is decomposed by immersing and agitating the biological tissue in a digestive juice containing digestive enzyme(s) (for example, refer to Patent Document 1).

Patent Document 1: PCT International Publication No. WO 2005/012480 Pamphlet

### Disclosure of Invention

However, when a biological tissue collected by using a biopsy instrument or the like is to be decomposed, mechanical loadings such as a shearing stress act on places where the biological tissue and the instrument have been in contact, and thus the biological tissue is denatured. Therefore, the decomposition is retarded even with exposure to a digestive juice, which leads to a problem in that it takes time to take inner cells out.
On the other hand, if the enzyme concentration in the digestive juice is increased, proteins in cell surfaces are degraded, which leads to a problem in that the health of cells is deteriorated.

The present invention takes the above problems into consideration, with an object of providing a method for decomposing biological tissue capable of decomposing a biological tissue in a short time and obtaining cells at a high yield without interfering with the health of cells obtained through the decomposition.

The present invention employs the following means in order to achieve the above object.
The present invention provides a method for decomposing biological tissue which decomposes a biological tissue under an action of a digestive enzyme, comprising: a decomposition starting step of starting the decomposition of the biological tissue by immersing the biological tissue in a digestive juice having the digestive enzyme mixed in a solvent; and an enzyme concentration reduction step of reducing a concentration of the digestive enzyme in the digestive juice at a predetermined timing after the start of the decomposition.

According to the present invention, when the decomposition of the biological tissue is started by the decomposition starting step, the biological tissue is decomposed due to the action of the digestive enzyme mixed in the solvent to release individual cells into the digestive juice. In this case, by mixing a relatively high concentration of the digestive enzyme into the solvent at the time of starting the decomposition, the decomposition of the biological tissue can be accelerated even in denatured cut faces. As a result, outflow of cells from a mass of the biological tissue can be started at a relatively early stage.

Then, at a timing when the cells separated from the biological tissue start floating in the digestive juice, by reducing the concentration of the digestive enzyme in the digestive juice by the enzyme concentration reduction step, a high concentration of the digestive enzyme can be prevented from acting on the floating cells, and the health of the cells can be maintained. Accordingly, the biological tissue can be decomposed in a short time and the yield of healthy cells can be increased.

In the above invention, the decomposition starting step may be performed using a digestive juice having a total amount of the digestive enzyme required for completing the decomposition of the biological tissue mixed in a solvent.
By so doing, the digestive enzyme to be supplied to the biological tissue does not exceed the total amount of the digestive enzyme required for completing the decomposition, and thus the biological tissue can be decomposed without interfering with the health of cells.

Moreover, in the above invention, the enzyme concentration reduction step may supply the digestive juice with the solvent.
By so doing, the concentration of the digestive enzyme in the digestive juice can be readily reduced.
Furthermore, in the above invention, the digestive juice may be additionally supplied with the solvent either continuously or stepwisely over a predetermined time within a period from start of the decomposition until completion of the decomposition.
By so doing, the concentration of the digestive enzyme in the digestive juice can be reduced either continuously or stepwisely, and the concentration of the digestive enzyme can be reduced in accordance with the reduction of the denatured biological tissue in cut faces of the surface layer of the biological tissue.

In the above invention, the enzyme concentration reduction step may add a digestive juice having a lower enzyme concentration than that of the digestive juice at the time of starting the decomposition.
Also by so doing, the concentration of the digestive enzyme in the digestive juice can also be readily reduced.

The above invention may also comprise: a total amount determination step of determining a total amount of the digestive enzyme required for completing the decomposition of the biological tissue; and an enzyme supply step of additionally supplying the digestive juice so that the determined total amount of the digestive enzyme can be supplied from start of the decomposition of the biological tissue until completion of the decomposition.
By so doing, since the total amount of the digestive enzyme determined by the total amount determination step is supplied from start of the decomposition until completion of the decomposition, more than necessary amount of the digestive enzyme is not added, thereby preventing the digestive enzyme having a high activity from acting on cells after the completion of the decomposition, and the health of the cells can be maintained.

In the above invention, the solvent may comprise either a Ringer's solution or a phosphate buffer solution.
By so doing, the biological tissue is decomposed by a mixed solution of the digestive enzyme with either a Ringer's solution or a phosphate buffer solution adjusted to near-neutral pH. Therefore, lowering in the survival rate of cells obtained after the decomposition can be prevented.

The present invention demonstrates effects enabling to decompose a biological tissue in a short time and to obtain cells at a high yield without interfering with the health of the cells obtained through the decomposition.

### Brief Description of Drawings

[FIG. 1] A flowchart showing a method for decomposing biological tissue according to one embodiment of the present invention.
[FIG. 2] A graph showing a timewise change of the normalized yield in the method for decomposing biological tissue of FIG. 1, with comparison to a conventional decomposition method.

### Explanation of Reference Signs

S1: Total amount determination step
S31: Additional supply step (Enzyme concentration reduction step)
S32: Agitation step (Decomposition starting step)

### Best Mode for Carrying Out the Invention

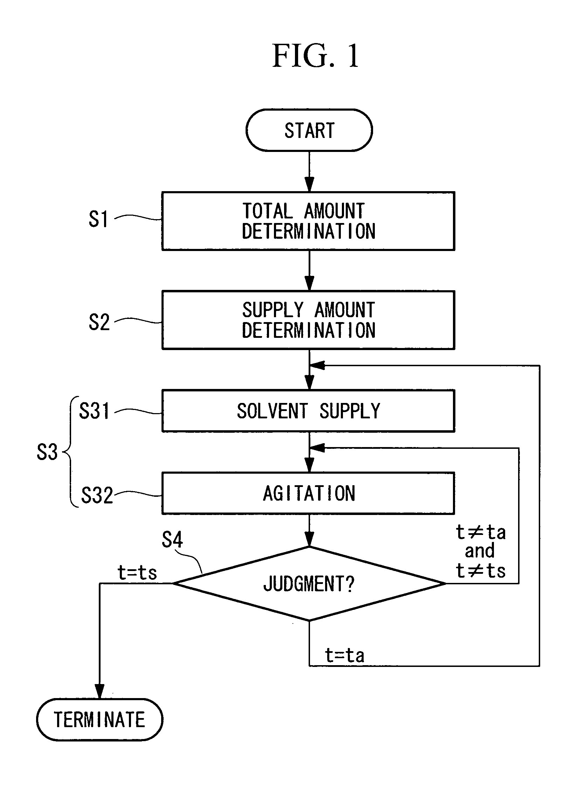

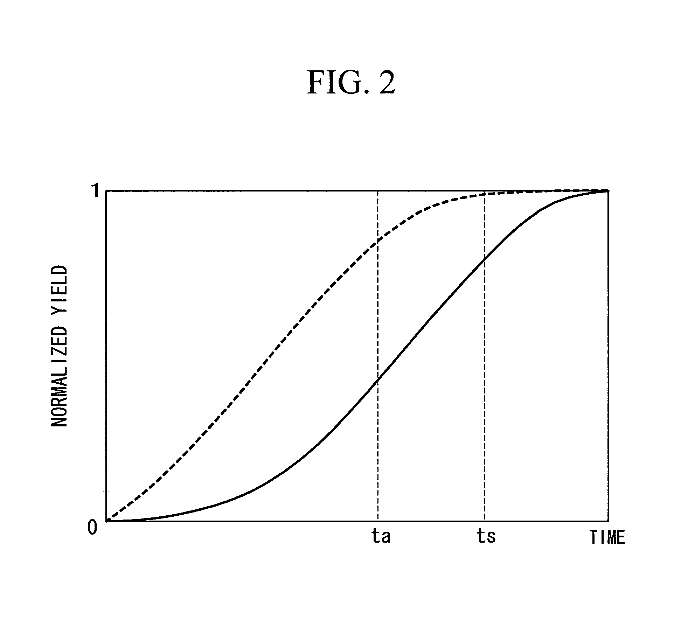

Hereunder is a description of a method for decomposing biological tissue according to one embodiment of the present invention, with reference to FIG. 1 and FIG. 2.
The method for decomposing biological tissue according to the present embodiment is, for example, a method for decomposing adipose tissue, which comprises step S1 to step S7 as shown in FIG. 1.

In the total amount determination step S1, an amount of a digestive juice required for completing the decomposition is determined based on the total weight or volume of an adipose tissue to be decomposed, so as to determine a total amount of the digestive enzyme and a total amount of a solvent such as a Ringer's solution or a phosphate buffer solution for constituting the digestive juice.
In the supply amount determination step S2, the total amount of the solvent determined in the total amount determination step S1 is divided into a plurality of portions to determine supply amounts for respective timings.

The decomposition step S3 comprises: a preparation step S31 (enzyme concentration reduction step) of preparing a digestive juice by mixing the digestive enzyme at the total amount determined in the total amount determination step S1 with the Ringer's solution at the supply amount determined in the supply amount determination step S2; and an agitation step S32 (decomposition starting step) of immersing and agitating the biological tissue in the prepared digestive juice.

In the judgment step S4, time elapsed from the start of the decomposition step S3 is measured to judge whether or not a predetermined time has elapsed. The elapsed time to be judged in the judgment step S4 is a time ta (a = 1 to n) for additionally supplying the Ringer's solution and a termination time of the decomposition (ts). If the judgment step S4 judges that the predetermined time ta has elapsed, the Ringer's solution at the supply amount that has been previously determined in the supply amount determination step S2 is additionally supplied to the digestive juice. Moreover, if it is judged that the termination time of the decomposition ts has elapsed, the decomposition is terminated.

According to thus constituted method for decomposing biological tissue according to the present embodiment, at the time of starting the decomposition of the biological tissue, the total amount of the digestive enzyme determined by the total amount determination step S1 and a part of the total amount of the Ringer's solution determined by the supply amount determination step S2 are mixed, and thereby the biological tissue is immersed in the digestive juice having a relatively high concentration of the digestive enzyme. Accordingly, the biological tissue immersed in the digestive juice of such a high concentration is readily digested. Particularly, by treating the biological tissue denatured by the mechanical loadings such as a shearing stress acting on places where the biological tissue and the instrument are in contact, with the digestive juice of high concentration, the decomposition of such a biological tissue can be accelerated.

Then, when the predetermined time ta has elapsed after the start of the decomposition, the Ringer's solution at a previously determined supply amount is additionally supplied to the digestive juice, and thus the enzyme concentration in the digestive juice can be reduced. That is to say, once the decomposition of the biological tissue in the denatured cut faces is completed, the biological tissue can be continuously decomposed even if the enzyme concentration in the digestive juice is reduced. Further, since the biological tissue is decomposed and individual cells start floating dispersingly in the digestive juice, cells can be kept from being exposed to a high concentration of the digestive enzyme by reducing the concentration of the digestive enzyme.

As a result, the disadvantage in which proteins in cell surfaces are damaged can be prevented and the collected cells can be maintained in a healthy state.
Further, by setting a high concentration of the digestive enzyme at the time of starting the decomposition, the decomposition immediately after the start of the decomposition is accelerated. Therefore, the entire time required for the decomposition of the biological tissue can be shortened.
Then, when the judgment step S4 judges that the termination time of the decomposition ts has elapsed, the decomposition is terminated.

FIG. 2 shows the relation between time required for the decomposition and normalized yield, with comparison to a conventional decomposition method which uses a digestive juice prepared by mixing the digestive enzyme and the Ringer's solution both at previously determined total amounts (solid line). In the graph, the broken line indicates the result obtained by the method for decomposing biological tissue according to the present embodiment. In this case, a half portion of the total amount of the Ringer's solution is supplied at the time of starting the decomposition, and the other half portion is additionally supplied after the predetermined time ta has elapsed.

According to this graph, the decomposition of the biological tissue immediately after the start of the decomposition was greatly increased by the digestive juice containing a high concentration of the digestive enzyme, as compared with a conventional case indicated by the solid line, showing that the decomposition was performed at an early stage. Moreover, after the Ringer's solution was additionally supplied when the predetermined time ta elapsed, the speed of the decomposition slowed down, but a same yield was achieved at a sufficiently early stage as compared with the conventional decomposition method indicated by the solid line, showing that the time for the decomposition can be greatly shortened.

In the present embodiment, the total amount of the digestive enzyme required for completing the decomposition was divided into two to be additionally supplied twice. However, instead of this, additional supply may be dividedly performed three times, or may be continuously performed within the range of the total amount of the Ringer's solution over a predetermined time.

Further, according to the method for decomposing biological tissue according to the present embodiment, the total amount of the digestive enzyme to be supplied to the digestive juice until completion of the decomposition is previously determined prior to the start of the decomposition based on the weight or volume of the biological tissue, and does not exceed the determined value. Accordingly, an advantage is also provided in which decomposed cells can be kept from being in contact with the digestive enzyme having a remaining high activity, to thereby improve the health of the obtained cells.

Moreover, in the present embodiment, either a Ringer's solution or a phosphate buffer solution adjusted to near-neutral pH was used as a solvent. By so doing, cells existing in the tissue can be collected without being damaged by pH during the decomposition. The matrix metalloproteinase used for the decomposition of the tissue requires metal ions for maintaining and improving its activity. By adding a salt containing such required metal ions to the solvent to be used, the activity can be improved and thus the yield of cells separated from the tissue can be improved.

## Claims

1. A method for decomposing biological tissue which decomposes a biological tissue under an action of a digestive enzyme, comprising:
a decomposition starting step of starting the decomposition of the biological tissue by immersing the biological tissue in a digestive juice having the digestive enzyme mixed in a solvent; and
an enzyme concentration reduction step of reducing a concentration of the digestive enzyme in the digestive juice at a predetermined timing after the start of the decomposition.

2. A method for decomposing biological tissue according to claim 1, wherein said decomposition starting step is performed using a digestive juice having a total amount of the digestive enzyme required for completing the decomposition of the biological tissue mixed in a solvent.

3. A method for decomposing biological tissue according to either one of claim 1 and claim 2, wherein said enzyme concentration reduction step supplies the digestive juice with the solvent.

4. A method for decomposing biological tissue according to claim 3, wherein the digestive juice is additionally supplied with the solvent either continuously or stepwisely over a predetermined time within a period from start of the decomposition until completion of the decomposition.

5. A method for decomposing biological tissue according to claim 1, wherein said enzyme concentration reduction step adds a digestive juice having a lower enzyme concentration than that of the digestive juice at the time of starting the decomposition.

6. A method for decomposing biological tissue according to claim 5, comprising: a total amount determination step of determining a total amount of the digestive enzyme required for completing the decomposition of the biological tissue; and
an enzyme supply step of additionally supplying the digestive juice so that the determined total amount of the digestive enzyme can be supplied from start of the decomposition of the biological tissue until completion of the decomposition.

7. A method for decomposing biological tissue according to any one of claim 1 through claim 6, wherein said solvent comprises either a Ringer's solution or a phosphate buffer solution.
